# EUROPÄISCHE PATENTSCHRIFT

(11) **EP 3 011 085 B1**
(45) Veröffentlichungstag und Bekanntmachung des Hinweises auf die Patenterteilung: **21.11.2018**
(21) Anmeldenummer: 14747289.8
(22) Anmeldetag: 13.06.2014
(51) Int. Cl.: D01F 2/08, C08L 5/00, C08B 37/00, D01F 9/00

(54) **POLYSACCHARIDFASER UND VERFAHREN ZU IHRER HERSTELLUNG**
POLYSACCHARIDE FIBRES AND METHOD FOR PRODUCING SAME
FIBRES EN POLYSACCHARIDES ET LEUR PROCÉDÉ DE FABRICATION

(30) Priorität: 17.06.2013 AT 4852013
(43) Veröffentlichungstag der Anmeldung: 27.04.2016
(73) Patentinhaber: Lenzing AG, 4860 Lenzing (AT)
(72) Erfinder: KRAFT, Gregor, A-4860 Lenzing (AT); KRONER, Gert, A-4863 Seewalchen (AT); RÖDER, Thomas, A-4840 Vöcklabruck (AT); FIRGO, Heinrich, A-4840 Vöcklabruck (AT)
(74) Vertreter: Hanemann, Otto
(86) Internationale Anmeldenummer: PCT/AT2014/000123
(87) Internationale Veröffentlichungsnummer: WO 2014/201482

(56) Entgegenhaltungen:
- WO-A1-2013/036968
- US-A- 3 600 379
- US-A1- 2011 200 776
- US-A1- 2013 087 938

## Beschreibung

Die vorliegende Erfindung betrifft ein Verfahren zur Herstellung von Polysaccharidfasern, die als faserbildendes Material α(1→3)-Glucan enthalten, sowie die daraus hergestellten Fasern und ihre Verwendung.

### Stand der Technik

Polysaccharide spielen als Materialien, die aus nachwachsenden Rohstoffen gewonnen werden können, eine immer größere Rolle. Eines der am häufigsten vorkommenden Polysaccharide ist die Cellulose. Baumwollfasern, die fast ausschließlich aus Cellulose bestehen, sind ein Beispiel für die Wichtigkeit von Polysacchariden. Aber auch aus anderen cellulosischen Rohstoffen gewonnene Materialien wie z. B. cellulosische Kunstfasern gewinnen immer mehr an Bedeutung.

Die Gattungsnamen "Viscose-Fasern" und "Modal-Fasern" wurden von der BISFA (Bureau for the International Standardization of Man-made Fibers) Cellulosefasern zugeteilt, die durch chemische Derivatisierung von Cellulose mithilfe von Natronlauge und Schwefelkohlenstoff (CS2) hergestellt werden.

Der Name "Modalfaser" ist ein generischer Begriff, der gemäß der Definition der BISFA für eine Cellulosefaser mit einer definierten hohen Nassfestigkeit und einem ebenfalls definierten hohen Nassmodul (d.h. die Kraft, welche benötigt wird, um die Faser in nassem Zustand um 5% zu dehnen) steht.

Bis heute hat sich jedoch nur ein Verfahren zur großtechnischen Herstellung von Fasern der Gattungen Viscose und Modal durchgesetzt und zwar das Viskose-Verfahren und Abwandlungen davon.

Wie dieses Verfahren ausgeführt wird, ist dem Fachmann grundsätzlich seit längerem aus vielen Patentschriften und sonstigen Publikationen bekannt. Ein Verfahren zur Herstellung von Modalfasern ist beispielsweise aus der AT 287.905 B bekannt.

CS2 hat in den bekannten Viskoseverfahren 2 wesentliche Aufgaben:
1. Reaktion mit Alkalicellulose zu einem Xanthogenat, das in alkalischer Lösung löslich ist
2. "Wirkung im Spinnbad"

Im Viskosespinnbad finden kolloidchemische (Koagulation des Natriumcellulosexanthogenats) und chemische (Zersetzung des Xanthogenats zu Hydratcellulose) Vorgänge parallel statt. Beide werden durch das eingesetzte CS2 beeinflusst.

Das Viscoseverfahren hat im Vergleich zu dem neuen ressourcen- und umweltschonende Lyocellverfahren einen schwerwiegenden Nachteil: Den Einsatz von großen Mengen an CS2 und Natronlauge. Dieses Problem betrifft die Herstellung von Modalfasern in noch größerem Maße. Im weiteren Text wird der Begriff Viscoseverfahren bzw. Viscosefaser für alle Viscoseverfahren und Abwandlungen, einschließlich des Modalverfahrens sowie damit hergestellten Fasern verwendet.

Der im Viskose-Verfahren bisher verwendete cellulosische Rohstoff ist Zellstoff, der vor allem aus Holz gewonnen wird. Trotz vieler Untersuchungen konnte bisher kein auf der Viscose-Technologie basierendes Verfahren entwickelt werden, mit dem der Einsatz von CS2 bzw. NaOH deutlich reduziert werden kann.

Die US 7,000,000 beschreibt Fasern, die durch Verspinnen einer Lösung von Polysacchariden, die im Wesentlichen aus Hexose-Wiederholungseinheiten bestehen, die über α(1→3)-glycosidische Bindungen verknüpft sind, erhalten werden. Diese Polysaccharide können hergestellt werden, indem eine wässrige Lösung von Saccharose mit Glucosyltransferase (GtfJ), isoliert aus Streptococcus salivarius, in Kontakt gebracht wird (Simpson et al. Microbiology, vol 41, pp 1451-1460 (1995)). "Im Wesentlichen" bedeutet in diesem Zusammenhang, dass innerhalb der Polysaccharidketten vereinzelt Fehlstellen auftreten können, an denen andere Bindungskonfigurationen auftreten. Diese Polysaccharide sollen für die Zwecke der vorliegenden Erfindung als "α(1→3)-Glucan" bezeichnet werden.

Die US 7,000,000 offenbart zunächst Möglichkeiten zur enzymatischen Herstellung von α(1→3)-Glucan aus Monosacchariden. Auf diese Weise können relativ kurzkettige Polysaccharide ohne Verlust an Monomerbausteinen hergestellt werden, da die Polymerketten aus den Monomerbausteinen aufgebaut werden. Im Gegensatz zur Herstellung kurzkettiger Cellulosemoleküle ist die Herstellung von α(1→3)-Glucan umso preisgünstiger, je kürzer die Polymerketten sind, da dann nur eine geringe Verweilzeit in den Reaktoren notwendig ist.

Gemäß der US 7,000,000 soll das α(1→3)-Glucan derivatisiert, bevorzugt acetyliert, werden. Das Lösungsmittel ist bevorzugt eine organische Säure, eine organische Halogenverbindung, ein fluorierter Alkohol oder eine Mischung aus solchen Komponenten. Diese Lösungsmittel sind teuer und aufwendig zu regenerieren.

Untersuchungen haben aber auch gezeigt, dass α(1→3)-Glucane in verdünnter Natronlauge (ca. 4 bis 5,5%) löslich sind.

Die WO 2013/052730 A1 offenbart Fasern mit α(1→3)-Glucan als faserbildende Substanz, die nach dem sogenannten Aminoxid-Verfahren mit NMMO als Lösungsmittel durch Verspinnen hergestellt wurden. Das Aminoxid-Verfahren ist grundsätzlich anders gestaltet als das Viscose- oder das Modal-Verfahren und erfordert eine völlig andere Produktionsanlage. Eine Viscose-Produktionsanlage kann nicht durch einfache Umbaumaßnahmen auf das Aminoxid-Verfahren umgestellt werden.

Die WO 2013/036968 A1 offenbart allgemein die Möglichkeit der Verwendung von α(1→3)-Glucan zur Herstellung von Regeneratcellulosefasern. Sie geht aber nicht näher darauf ein, welches der vielen verschiedenen Herstellverfahren für cellulosische Regeneratfasern gemeint sein soll.

Die US 3,600,379 offenbart ein modifiziertes Verfahren zur Herstellung einer Spinnlösung nach einem Viskoseverfahren, in dem mit geringeren Konzentrationen an NaOH und CS2 gearbeitet wird, und mit denen gute Filterwerte erreicht werden sollen. Aber in US 3,600,379 wird der nachfolgende Spinnprozess mit dieser Spinnlösung nicht einmal beschrieben, geschweige denn darauf eingegangen, ob bzw. welche Qualität von Cellulosefasern damit erhalten werden soll.

### Aufgabe

Die Aufgabe bestand gegenüber diesem Stand der Technik daher darin, ein alternatives Verfahren zur Herstellung von Polysaccharid-Faser zur Verfügung zu stellen, mit dem die Einsatzmenge von CS2 und Natronlauge je hergestellter Einheit Faser deutlich reduziert werden kann.

### Beschreibung der Erfindung

Die Lösung der oben beschriebenen Aufgabe besteht in einem neuen Verfahren zur Herstellung einer Polysaccharid-Faser, deren faserbildende Substanz α(1→3)-Glucan ist, wobei das Verfahren ein modifiziertes Viscoseverfahren ist. Damit kann eine viscoseartige Faser hergestellt werden,. Maximal werden 30% CS2 bezogen auf die faserbildende Substanz eingesetzt, bevorzugt weniger als 25% CS2 und besonders bevorzugt weniger als 15% CS2. Bevorzugt ist eine Einsatzmenge zwischen 5 und 30 Gewichtsprozent CS2, berechnet auf faserbildendes Material, bevorzugt zwischen 5 und 25 %, besonders bevorzugt zwischen 5 und 15%. Eine solche Faser soll für die Zwecke der vorliegenden Erfindung ebenfalls als Viscose-Faser bezeichnet werden, obwohl sie anstatt Cellulose ein anderes faserbildendes Polysaccharid, nämlich das α(1→3)-Glucan, enthält.

Überraschenderweise konnte festgestellt werden, dass CS2 in diesem modifizierten Prozess nicht wie im Falle der Cellulose für die Lösung des Polysaccharids in Natronlauge benötigt wird, sondern ausschließlich für die Verlangsamung der Fadenbildung im Spinnbad.

Die NaOH-Konzentration in der Spinnlösung soll erfindungsgemäß zwischen 4,0 und 5,5 Gew.-%, berechnet auf die Gesamtmenge der Spinnlösung betragen. Außerhalb dieses Bereichs ist die Löslichkeit des Glucans nicht ausreichend.

Für die Zwecke der vorliegenden Erfindung soll der Begriff "Faser" sowohl Stapelfasern mit definierter Schnittlänge als auch Endlosfilamente umfassen. Sämtliche im Folgenden beschriebenen Prinzipien der Erfindung gelten grundsätzlich sowohl für Stapelfasern als auch für Endlosfilamente.

Der Einzelfasertiter der erfindungsgemäßen Fasern kann zwischen 0,1 und 10 dtex betragen. Bevorzugt ist er zwischen 0,5 und 6,5 dtex und besonders bevorzugt zwischen 0,9 und 6,0 dtex. Im Falle von Stapelfasern beträgt die Schnittlänge üblicherweise zwischen 0,5 und 120 mm, bevorzugt zwischen 20 und 70 mm und besonders bevorzugt zwischen 35 und 60 mm. Im Falle von Endlosfilamenten beträgt die Anzahl der Einzelfilamente im Filamentgarn zwischen 50 und 10.000, bevorzugt zwischen 50 und 3.000.

Das α(1→3)-Glucan kann hergestellt werden, indem eine wässrige Lösung von Saccharose mit Glucosyltransferase (GtfJ), isoliert aus Streptococcus salivarius, in Kontakt gebracht wird (Simpson et al. Microbiology, vol 41, pp 1451-1460 (1995)).

In einer bevorzugten Ausführungsform des erfindungsgemäßen Verfahrens besteht das α(1→3)-Glucan zu mindestens 90 % aus Hexose-Einheiten und mindestens 50 % der Hexose-Einheiten sind durch α(1→3)-glycosidische Bindungen verknüpft.

Das Verfahren zur Herstellung der erfindungsgemäßen Faser besteht aus folgenden Schritten:
1. Herstellung einer α(1→3)-Glucan Lösung in verdünnter Natronlauge
2. Zugabe und Einmischen von CS2, eine kurze Nachreife, Filtration und Entlüftung der Spinnlösung
3. Ausspinnen der α(1→3) Glucanhaltigen Spinnlösung durch eine Düse in ein schwefelsaures Spinnbad, Verstreckung der Fasern und Nachbehandlung.

Die Konzentration der faserbildenden Substanz in der Spinnlösung kann zwischen 4 und 18 Gew.-% betragen, bevorzugt sind 4,5 bis 15 Gew.-%.

Der Polymerisationsgrad des im erfindungsgemäßen Verfahren eingesetzten α(1→3) Glucans, ausgedrückt als Gewichtsmittel DP_{w}, kann zwischen 200 und 2000 liegen; bevorzugt sind Werte zwischen 500 und 1000.

Gegenstand der vorliegenden Erfindung ist auch eine Viscose- oder ModalFaser, die Cellulose und α(1→3)-Glucan enthält.

In einer bevorzugten Ausführungsform besteht das α(1→3)-Glucan der erfindungsgemäßen Viscose-Faser zu mindestens 90 % aus Hexose-Einheiten und mindestens 50 % der Hexose-Einheiten sind durch α(1→3)-glycosidische Bindungen verknüpft.

Ebenfalls Gegenstand der vorliegenden Erfindung ist die Verwendung der erfindungsgemäßen Fasern zur Herstellung von verschiedensten trocken- und nass gelegten Papieren, Vliesstoffen, Hygieneartikeln wie Tampons, Slipeinlagen und Windeln und sonstigen Vliesstoffen, insbesondere absorbierenden Nonwovens-Produkten, aber auch von textilen Erzeugnissen wie Garnen, Geweben, Gestricken oder Gewirken.

Im Folgenden wird die Erfindung anhand von Beispielen beschrieben. Die Erfindung ist jedoch ausdrücklich nicht auf diese Beispiele beschränkt, sondern umfasst auch alle anderen Ausführungsformen, die auf dem gleichen erfinderischen Konzept beruhen.

### Beispiele

Der Polymerisationsgrad der α(1→3)-Glucane wurde mittels GPC in DMAc/LiCI ermittelt. Im Folgenden wird stets das Gewichtsmittel des Polymerisationsgrades (DP_{w}) angegeben.

### Beispiel 1:

Eine wässrige Glucanlösung, enthaltend 9,1% α(1→3)-Glucan mit einem DP_{w} von 800 sowie 4,5 Gew.-% NaOH wurde mit 7,5% CS2 (Gewichtsprozent berechnet auf faserbildendes Material) umgesetzt. Die so hergestellte Viskose enthält9 Gew.-% faserbildendes Material, 4,5 Gew.-% NaOH und 0,57 Gew.-% Schwefel. Die Lösung wurde mittels einer Spinndüse in ein Regenerierbad, enthaltend 100 g/l Schwefelsäure, 330 g/l Natriumsulfat und 35 g/l Zinksulfat, gesponnen. Die Spinndüse hatte 1053 Löcher mit 50µm Durchmesser. Der Viskosespinnlösung wurden 2,5 Gew.-% eines stickstoffhaltigen Hilfsmittels (Leomin AC80) zugesetzt. Zur Erzielung einer entsprechenden Faserfestigkeit erfolgt eine Verstreckung im Zweitbad (92 C, 15 g/l H2S04) von ca. 75%. Die Abzugsgeschwindigkeit beträgt 30 m/min.

Die Eigenschaften der erhaltenen Fasern sind in der Tabelle 1 angegeben:

### Beispiel 2:

Eine wässrige Glucanlösung, enthaltend 11% α(1→3)-Glucan mit einem DP_{w} von 1000 sowie 4,8 Gew.-% NaOH wurde mit 15% CS2 (Gewichtsprozent berechnet auf faserbildendes Material) umgesetzt. Die so hergestellte Viskose enthält 10,8 Gew.-% faserbildendes Material, 4,7 Gew.-% NaOH und 1,37 Gew.-% Schwefel. Die Lösung wurde mittels einer Spinndüse in ein Regenerierbad, enthaltend 100 g/l Schwefelsäure, 330 g/l Natriumsulfat und 45 g/l Zinksulfat, gesponnen. Die Spinndüse hatte 1053 Löcher mit 50µm Durchmesser. Der Viskosespinnlösung wurden 3 Gew.-% einen stickstoffhaltigen Hilfsmittels zugesetzt. Zur Erzielung einer entsprechenden Faserfestigkeit erfolgt eine Verstreckung im Zweitbad (92 C, 15 g/l H2S04) von ca. 75%. Die Abzugsgeschwindigkeit beträgt 25 m/min. Die Eigenschaften der erhaltenen Fasern sind in der Tabelle 1 angegeben:

### Beispiel 3:

Eine wässrige Glucanlösung, enthaltend 12,5% α(1→3)-Glucan mit einem DP_{w} von 800 sowie 4,4 Gew.-% NaOH wurde mit 12% CS2 (Gewichtsprozent berechnet auf faserbildendes Material) umgesetzt. Die so hergestellte Viskose enthält 12,3 Gew.-%% faserbildendes Material, 4,3 Gew.-% NaOH und 1,24 Gew.-% Schwefel. Die Lösung wurde mittels einer Spinndüse in ein Regenerierbad, enthaltend 90 g/l Schwefelsäure, 330 g/l Natriumsulfat und 45 g/l Zinksulfat, gesponnen. Die Spinndüse hatte 1053 Löcher mit 50µm Durchmesser. Der Viskosespinnlösung wurden 1% eines stickstoffhaltigen Hilfsmittels zugesetzt. Zur Erzielung einer entsprechenden Faserfestigkeit erfolgt eine Verstreckung im Zweitbad (92°C, 15 g/l H2S04) von ca. 75%. Die Abzugsgeschwindigkeit beträgt 27 m/min. Die Eigenschaften der erhaltenen Fasern sind in der Tabelle 1 angegeben:
Die Eigenschaften der erhaltenen Fasern sind in der Tabelle 1 angegeben:

**Tabelle 1**

| Beispiel | Titer dtex | FFk cN/tex | FDk % |
|---|---|---|---|
| Bsp 1 | 1,7 | 17,3 | 19,1 |
| Bsp 2 | 1,3 | 23,4 | 16,3 |
| Bsp 3 | 1,5 | 21,8 | 18,1 |

| | | | |
|---|---|---|---|
| FFk Faserfestigkeit konditioniert FDk Faserdehnung konditioniert | | | |

## Patentansprüche

1. Verfahren zur Herstellung einer Polysaccharid-Faser, deren faserbildende Substanz α(1→3)-Glucan ist, **dadurch gekennzeichnet, dass** das Verfahren ein modifiziertes Viscoseverfahren ist, wobei maximal 30 Gew.-% CS2 bezogen auf faserbildende Substanz eingesetzt werden.

2. Verfahren nach Anspruch 1, wobei das α(1→3)-Glucan zu mindestens 90 % aus Hexose-Einheiten besteht und mindestens 50 % der Hexose-Einheiten durch α(1→3)-glycosidische Bindungen verknüpft sind.

3. Verfahren gemäß den vorhergehenden Ansprüchen, wobei die Faser eine Stapelfaser oder ein Endlosfilament ist.

4. Verfahren gemäß den vorhergehenden Ansprüchen, wobei , bevorzugt weniger als 25 Gew.-% CS2, besonders bevorzugt weniger als 15 Gew.-% CS2 bezogen auf faserbildende Substanz eingesetzt werden.

5. Polysaccharid-Faser, hergestellt nach einem modifizierten Viscoseverfahren, **dadurch gekennzeichnet, dass** die faserbildende Substanz α(1→3)-Glucan ist, wobei in dem modifizierten Viscoseverfahren maximal 30 Gew.-% CS2 bezogen auf faserbildende Substanz eingesetzt werden.

6. Faser nach Anspruch 5, wobei das α(1→3)-Glucan zu mindestens 90 % aus Hexose-Einheiten besteht und mindestens 50 % der Hexose-Einheiten durch α(1→3)-glycosidische Bindungen verknüpft sind.

7. Faser gemäß den Ansprüchen 5 oder 6, wobei die Faser eine Stapelfaser oder ein Endlosfilament ist.

8. Verwendung der Faser nach Anspruch 5 zur Herstellung von textilen Erzeugnissen wie Garnen, Geweben, Gestricken oder Gewirken.

9. Verwendung der Faser nach Anspruch 5 zur Herstellung von Vliesstoffen, Hygieneartikeln, insbesondere Tampons, Slipeinlagen und Windeln und sonstigen, absorbierenden Nonwovens-Produkten und Papieren.

10. Verwendung gemäß den Ansprüchen 8 oder 9, wobei die Faser eine Stapelfaser oder ein Endlosfilament ist.

## Claims

1. A method for the production of a polysaccharide fiber whose fiber-forming substance is α(1→3)-glucan, **characterized in that** the method is a modified viscose process, wherein CS₂ is added to the α(1→3)-glucan-containing sodium hydroxide solution.

2. The method as claimed in claim 1, wherein at least 90% of the α(1→3)-glucan are hexose units and at least 50% of the hexose units are linked via α(1→3)-glycosidic bonds.

3. The method as claimed in any of the preceding claims, wherein the fiber is a staple fiber or a continuous filament.

4. The method as claimed in any of the preceding claims, wherein no more than 30% by weight of CS₂, related to the fiber-forming substance, are used, preferably less than 25% by weight of CS₂, and more preferably less than 15% by weight of CS₂.

5. A polysaccharide fiber produced using a modified viscose process, wherein CS₂ is added to the α(1→3)-glucan-containing sodium hydroxide solution, **characterized in that** the fiber-forming substance is α(1→3)-glucan.

6. The fiber as claimed in claim 5, wherein at least 90% of the α(1→3)-glucan are hexose units and at least 50% of the hexose units are linked via α(1→3)-glycosidic bonds.

7. The fiber as claimed in claims 5 or 6, wherein the fiber is a staple fiber or a continuous filament.

8. A use of the fiber as claimed in claim 5 for the production of textile products such as yarns, woven fabrics, or knitted fabrics.

9. A use of the fiber as claimed in claim 5 for the production of nonwovens, hygiene articles, particularly tampons, panty liners, and diapers, and of other, absorbent nonwoven products and papers.

10. The use as claimed in claims 8 or 9, the fiber being a staple fiber or a continuous filament.

## Revendications

1. Procédé pour la production d'une fibre polysaccharidique dont la substance formant les fibres est l'α(1→3)-glucane, **caractérisé en ce que** ledit procédé est un procédé Viscose modifié usant au maximum 30 % en poids de CS2 par rapport à la substance formant les fibres.

2. Procédé selon la revendication 1, l'α(1→3)-glucane étant composé d'au moins 90 % d'unités d'hexose, au moins 50 % des unités d'hexose étant reliées par des liaisons glycosidiques α(1→3).

3. Procédés selon les revendications précédentes, ladite fibre étant une fibre discontinue ou un filament continu.

4. Procédés selon les revendications précédentes, utilisant de préférence moins de 25 % en poids de CS2, et tout particulièrement moins de 15 % en poids de CS2, par rapport à la substance formant les fibres.

5. Une fibre polysaccharidique, produite selon un procédé Viscose modifié, **caractérisé en ce que** la substance formant les fibres est l'α(1→3)-glucane, ledit procédé Viscose modifié usant au maximum 30 % en poids de CS2 par rapport à la substance formant les fibres.

6. Une fibre suivant la revendication 5, l'α(1→3)-glucane étant composé d'au moins 90 % d'unités d'hexose, au moins 50 % des unités d'hexose étant reliées par des liaisons glycosidiques α(1→3).

7. Une fibre suivant les revendications 5 ou 6, la fibre étant une fibre discontinue ou un filament continu.

8. Utilisation de la fibre selon la revendication 5 pour la production de produits textiles tels que les fils, les tissus ou les étoffes de bonneterie.

9. Utilisation de la fibre selon la revendication 5 pour la production de non-tissés, de produits sanitaires, en particulier les tampons, les serviettes hygiéniques, les couches et autres, les produits non-tissés absorbants et les papiers.

10. Utilisation de la fibre selon les revendications 8 ou 9, ladite fibre étant une fibre discontinue ou un filament continu.
